(19) Europäisches Patentamt / European Patent Office / Office européen des brevets

(11) **EP 4 134 004 A1**

(12) **EUROPEAN PATENT APPLICATION**

(43) Date of publication:
**15.02.2023 Bulletin 2023/07**

(21) Application number: **21190800.9**

(22) Date of filing: **11.08.2021**

(51) International Patent Classification (IPC):
**A61B 5/1486** (2006.01)    **G01N 27/327** (1990.01)
**G01N 27/403** (1990.01)    **G01N 27/413** (1990.01)
**G01N 27/48** (1968.09)    **G01N 27/49** (1990.01)

(52) Cooperative Patent Classification (CPC):
**G01N 27/4035; A61B 5/14532; A61B 5/1477;**
**A61B 5/1486;** A61B 5/14507; A61B 5/14542;
G01N 27/3271; G01N 27/413

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Roche Diabetes Care GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR**
  **HR HU IE IS IT LI LT LU LV MC MK MT NL NO PL**
  **PT RO RS SE SI SK SM TR**

(72) Inventor: **Pötschke, Markus**
  **68305 Mannheim (DE)**

(74) Representative: **Grünecker Patent- und**
  **Rechtsanwälte**
  **PartG mbB**
  **Leopoldstraße 4**
  **80802 München (DE)**

(54) **REGULATION OF A TWO-ELECTRODE ANALYTE SENSOR**

(57) The invention relates to a system, a sensor and a method for measuring an analyte concentration using an electrochemical analyte sensor, the analyte sensor comprising a first electrode and a second electrode, the first electrode being configured to react with the analyte for generating an electrical signal, wherein the method comprises: applying (201) a modulated voltage signal between the first electrode and the second electrode, determining (202) a current signal in response to the applied modulated voltage signal, determining (203) an electric potential working point of the analyte sensor based on the determined current signal, operating (204) the analyte sensor at the determined electric potential working point, and measuring (205) the analyte concentration based on the electrical signal generated by the first electrode.

FIG. 2

**Description**

State of the art

The invention relates to an electro-chemical analyte sensor and to a method of operating an electrochemical analyte sensor for measuring an analyte concentration.

Electrochemical analyte sensors exploit the fact that certain electrochemical reactions of an analyte to be detected or to be measured can generate electrical signals, e.g. electric currents or electric potentials, that can correlate with the concentration of the analyte to be detected, e.g. that are directly or indirectly proportional to the concentration of the analyte.

For example, a potentiometric electrochemical analyte sensor can generate electric potentials that can exhibit a logarithmic dependence on the concentration of an analyte to be detected.

For example, an amperometric electrochemical analyte sensor can generate an electric current that can flow between electrodes of the sensor that can be proportional to the concentration of the analyte to be measured.

Such analyte sensors are, for example, commonly used for measuring glucose levels, and in particular can be part of in-vivo continuous glucose monitoring systems that measure glucose levels in the blood and/or in interstitial fluids of patients suffering from diabetes.

However, current electrochemical analyte sensors suffer from numerous drawbacks. For example, electrodes of current analyte sensors can exhibit significant material wear and can leak undesired chemicals or materials into their environment.

Furthermore, the reference point for measurements of current analyte sensors, e.g. a reference electric potential, can shift or drift over time in unexpected ways, thereby significantly degrading the accuracy, robustness and reliability of analyte measurements.

In addition, the structure and design of current electrochemical analyte sensors are rather complex and intricate, and require complicated and costly manufacturing steps.

Problem

It is therefore an objective of the present invention to provide a method and means for obtaining more robust, more accurate and more reliable analyte measurements, e.g. glucose measurements, using an electrochemical analyte sensor.

It is further an objective of the present invention to provide an improved electrochemical analyte sensor.

In particular, it is an objective to provide a simplified, more compact, more cost-effective, more precise and more reliable electrochemical analyte sensor.

Solution

According to the present invention, said objectives are achieved by a method, an analyte sensor and a system according to the independent claims.

Advantageous embodiments and further developments are the subject matter of the subclaims.

Further advantageous embodiments and further developments are disclosed throughout the specification.

For example, a method for measuring an analyte concentration using an electrochemical analyte sensor may comprise an analyte sensor comprising a first electrode and a second electrode, wherein the first electrode can be configured to electrochemically react with the/an analyte for generating an electrical signal, e.g. a current signal, and wherein the method may comprise one, some or all of the following exemplary steps:

- applying a modulated voltage signal between the first electrode and the second electrode,

- determining a current signal in response to the applied modulated voltage signal,

- determining an electric potential working point of the analyte sensor, e.g. an electric potential working point of the first electrode, based on the determined current signal,

- operating the analyte sensor, e.g. the first electrode, at the determined electric potential working point, and

- measuring the analyte concentration based on the electrical signal generated by the first electrode, said electrical signal being generated in response to the electrochemical reaction from the first electrode operating at the determined electric potential working point and reacting with the analyte to be measured at the first electrode.

Herein, the determined electric potential working point can also be understood as electric reference potential point that can serve as a reference for the analyte concentration measurements of the analyte sensor.

Herein the step of determining an electric potential working point of the analyte sensor based on the determined current signal may be understood as setting or identifying or reaching or obtaining or regulating an electric potential working point based on the determined current signal.

Furthermore, the step of determining an electric potential working point of the analyte sensor based on the determined current signal may comprise or be understood as setting or identifying or reaching or obtaining or regulating an electric potential working point range based on the determined current signal or as selecting or setting an electric potential working point from an electric potential working point range that has been set or identified or obtained from the determined current signal.

The above and herein exemplary described means and method steps have numerous advantages over the current means and techniques for measuring an analyte concentration using an electrochemical analyte sensor.

For example, it has been unexpectedly and surprisingly found that an analyte sensor operating at an electric potential working point or at/within an electric potential working point range that has been obtained or set or determined using a modulated voltage signal applied between the first electrode and the second electrode works stably, without sensor drift or unexpected changes in a/the reference potential.

This is inter alia due to the fact that the herein exemplary described means and steps of applying a modulated voltage signal between the first electrode and the second electrode of the analyte sensor allow the more precise determination of an electric potential working point or electric potential working point range at which an electrode, i.e. the first electrode that can act as a working electrode, can work/operate at an electric potential that is close to the saturation region of the potential.

Said electric potential working point or electric potential working point range determined by the application of a modulated voltage signal between the first electrode and the second electrode can inter alia ensure that the electrical signal, e.g. a current signal, generated at the first electrode from the electrochemical reaction between the first electrode and the analyte to be measured is positively correlated to the concentration of the analyte to be measured, e.g. is directly proportional to the concentration of the analyte, wherein the proportional factor is constant and does not change over time.

Furthermore, it can be ensured that the generated electrical signal and therefore the determination of analyte concentration to be measured is not significantly affected by changes, e.g. drifts, of a/the reference electrical potential of the analyte sensor.

This technical finding overcomes the current prejudice that controllable, stable and drift-free or drift-compensated analyte sensors require setups with three or more electrodes with one or more dedicated reference electrodes.

Stated differently, the herein described means and method step also allow dispensing with the need for a dedicated reference electrode, in particular reference electrodes that are prone to undesired material wear and that can leak undesired material into their environments, such as, for example, Ag/AgCl (silver / silver chloride) reference electrodes. This can inter alia improve the lifetime of an analyte sensor.

The/said modulated voltage signal that can be applied between the first electrode and the second electrode can be applied in time-discrete steps.

Herein a time-discrete step can inter alia also be understood as a time interval.

Furthermore, at/during each time step or at/during each time interval, a modulation of the voltage signal can be applied.

For example, an exemplary modulated voltage signal $V(t)$ that can be applied by a controller between a/the first electrode and a/the second electrode of an exemplary analyte sensor may be exemplary defined by the following equation.

$$V(t) = V_{cont}(t) + \hat{V} \sin(2\pi f t) \qquad (1)$$

Therein, t is a/the time parameter, wherein t can be a continuous (analog) or a discrete (digital) time parameter, e.g. in case of t being a discrete (digital) time parameter t can, for example, be represented by a time step or time interval $t_k$, with index k being a natural number, $V_{cont}(t)$ is an exemplary voltage applied / outputted by a controller at time t or at step or time interval $t_k$, $\hat{V}$ is an exemplary voltage amplitude also set/controlled by a/the controller, sin() is the sinus function, and $f$ is a frequency, i.e. a modulation frequency.

The controller can be analog or digital, e.g. can be a micro controller or another digital control device.

When using a digital controller, a/the to be applied (analog) modulated voltage signal V(t) may be generated from digital controller voltage outputs by means of a digital-to-analog converter (DAC) or by means of using pulse-width-modulation (PWM) in combination with an analog low pass filter.

For example, after calculating the sum of the exemplary voltage modulation terms in the above-identified equation (1), the calculated sum can be passed to a digital-to-analog converter (DAC) or to a component applying a pulse-width-modulation (PWM) with a low pass filter to generate an/the (analog) modulated voltage signal V(t) to be applied between a/the first electrode and a/the second electrode of an exemplary analyte sensor.

Furthermore, it is inter alia also conceivable to periodically modulate a resistance or drive a discharge current by other means through the analyte sensor (or additional) capacity and thereby also modulate the voltage signal. This, for example, could be beneficial in combination with energy harvesting or to reduce the power consumption.

The above-identified modulated voltage signal is exemplary only and other modulations are conceivable too. For example, it is conceivable that the voltage signal is modulated not at every time step, but only at predetermined time steps or that the voltage signal is modulated continuously or quasi-continuously.

Furthermore, an exemplary modulated voltage signal V(t) to be applied between a/the first electrode and a/the second electrode of an exemplary analyte sensor may itself also be generated using pulse-width-modulation (PWM).

An exemplary sampling time or time interval, $t_k$, can be in the order of $0.01/f$, e.g. 1/16 Hz to 1 Hz.

Such an exemplary sampling time or time interval provides a sufficient signal resolution, while also being energy efficient and allowing an optimized power consumption of the analyte sensor.

Furthermore, it can allow the avoidance of signal interference from possible polarization frequency signal terms that may be caused by possible resonating components in the environment of the analyte sensor, e.g. stemming from a possibly polarized electrolyte solution.

An exemplary frequency $f$ may lie around a few cycles per minute, e.g. 1/min - 10/min, but can also be higher.

Furthermore, said exemplary frequency $f$ may also be set such as to avoid possible interference with frequency ranges from other resonators that might be present in the environment of the analyte sensor.

The exemplary voltage amplitude $\hat{V}$ can lie in the order of a few mV, e.g. 1 to 10 mV, preferably lower than 25 mV.

This can inter alia ensure a sufficient signal-to-noise ratio.

As a consequence of the exemplary modulated voltage signal applied between the first electrode and the second electrode of the analyte sensor, a modulated current signal $I(t)$ can be generated.

Hence, a/the current signal $I(t)$ of the analyte sensor in response to the applied modulated voltage signal and/or in response to the electrical signal generated at the first electrode from the reaction with the analyte can be determined / measured.

The measuring and/or processing of the current signal can be carried out in an analog and/or digital manner. For example, it is conceivable to use an analog-digital-converter (ADC) to sample the current signal for subsequently digital processing, which can be preferred for most applications. However, it is also possible to process the current signal in an analog circuit only.

The amplitude $\hat{I}$ of said generated current signal, for example, can be measured / determined as follows.

A Discrete Fourier Transformation (DTF) with an implementation of a Fast Fourier Transformation (FFT) can be applied. For example, if a single sine, as in the example according to equation 1 above, or a single cosine is applied as modulation signal, only one frequency of the Fourier spectrum is of particular relevance and determination of the sought-after signal amplitude $\hat{I}$ can be simplified to:

$$\hat{I}^2 = \left( \sum_k I(t_k) * sin(2\pi f t_k) \right)^2 + \left( \sum_k I(t_k) * cos(2\pi f t_k) \right)^2 \qquad (2)$$

Alternatively, a lock-in-amplifier can be used for processing an/the analog current signal and the modulation signal to isolate the signal component (e.g. $\hat{I} sin(2\pi f t_k + \Delta\Phi)$ with $\Delta\Phi$ being a phase shift) and to return a voltage that is proportional to $\hat{I}$ as output.

For sinusoidal modulation signals, such as in the example according to equation 1 above, the task can be simplified to measuring the signal power or maximum amplitude of the output of a narrow band pass filter centered on the modulation frequency.

Furthermore, the average current signal $\langle I(t) \rangle$ can be measured / determined.

Again such a measurement or determination can be carried out in an analog or digital manner.

For example, in the case of a digital solution a smoothing filter, e.g. a moving average filter, can be applied to the sampled current signal data, whereas in the case of an analog solution in an analog circuit a low pass filter can be used.

The step of determining the electric potential working point (or electric potential working point range) of the analyte sensor based on the determined current signal $I(t)$, wherein said current signal has been generated in response to the applied modulated voltage signal $V(t)$ can exemplary comprise the following regulation step:

- A regulation of the applied modulated voltage signal $V(t)$ such that the ratio of the determined amplitude $\hat{I}$ of the current signal at the first electrode to the determined average current $\langle I(t) \rangle$ signal falls within a predetermined range, e.g. a range set by a maximum threshold, $max_{threshold}$, and a minimum threshold, $min_{threshold}$, to determine / set / identify / reach or obtain an electric potential working point of the first electrode.

Stated differently, the level of the applied modulated voltage signal $V(t)$ can be regulated such that the following condition is fulfilled.

$$min_{threshold} \leq \frac{\hat{I}}{\langle I(t) \rangle} \leq max_{threshold} \qquad (3)$$

The regulation of the applied modulated voltage signal $V(t)$ can, for example, inter alia be carried out using a proportional-integral controller, e.g. a proportional-integral controller with $\log \frac{\hat{I}}{\langle I(t) \rangle}$ as input.

For example, the maximum threshold, $max_{threshold}$, may be equal or close to 0.05 and the minimum threshold, $min_{threshold}$, may be equal or close to 0.01.

For example, it may be possible that a/the controller for regulating the applied modulated voltage signal $V(t)$, i.e. a/the proportional-integral controller can be set / configured such that the condition according to equation 3 is fulfilled. For example, a/the proportional-integral controller can be set / configured such that $\frac{\hat{I}}{\langle I(t) \rangle}$ is set to a fixed value, e.g. 0.01 or 0.02.

For example, a digital proportional integral controller with a digitally computed input $\log \frac{\hat{I}}{\langle I(t) \rangle}$ may be used. However, also an analog proportional integral controller, with an analog input $\log \frac{\hat{I}}{\langle I(t) \rangle}$ can be used as well, wherein the analog proportional integral controller can inter alia comprise various operation amplifier circuits.

In any case, a negative reference value, e.g. log(0.01) or other value, can be selected / set as a reference value for the controller. This can inter alia reduce signal noise and improve control loop stability.

For example, when applying a voltage level $V_L$ of the applied modulated voltage signal $V(t)$ which satisfies the above-identified condition, an electric potential working point $E_{wp}$ for the analyte sensor, i.e. for the first electrode / working electrode / anode, can be reached / set / identified / obtained that lies close to a (local) saturation point or saturation region of the current-potential relation of the analyte sensor.

Herein, the expression close to a (local) saturation point or saturation region may refer to the case of the electric potential of the first electrode / working electrode / anode being within a desired or predetermined percentage range, e.g. within 20% or within 10% or less, with respect to an electric potential of the first electrode / working electrode / anode in the saturation region / saturation plateau of the current-potential relation of the analyte sensor.

Herein, $V_L$ can inter alia be understood as an average or mean of the applied modulated voltage signal $V(t)$, and wherein, for example, when the applied modulated voltage signal is a mean-free signal, e.g. a sine signal, the controller output voltage $V_{cont}$ can converge to $V_L$, after possible transient signals of the controlled system have decayed.

In other words, for example, after an initialization time of the analyte sensor, $V_{cont}$ can be considered an estimator for $V_L$.

Using the above exemplary described step of regulating the applied modulated voltage signal $V(t)$ such that, for example, the determined current signal $I(t)$ fulfills a certain condition, e.g. the above-identified condition according to equation 3, the electric potential at the first electrode / working electrode / anode is driven towards the desired electric potential working point $E_{wp}$.

Hence, applying a modulated voltage signal between the first electrode / working electrode / anode and the second electrode / cathode of the analyte sensor and determining a current signal in response to the applied modulated voltage signal, can allow to implicitly determine / set / reach / identify / obtain the desired electric potential working point $E_{wp}$ of the analyte sensor.

This is inter alia also reflected in the following relation.

$$V_L = E_A + E_C \qquad (4)$$

, wherein $E_C$ is the electric potential of/at the second electrode of the analyte sensor, wherein the index $C$ exemplary denotes the second electrode as cathode, $E_A$ is the electric potential of/at the second electrode / working electrode of the analyte sensor, wherein the index $A$ exemplary denotes the second electrode as anode.

Herein, the electric potential $E_C$ of the second electrode / cathode may inter alia be known or determined from factory calibrations or from calibrations during use / operation of the analyte sensor.

As indicated above, when applying a/the modulated voltage signal between the first electrode / working electrode / anode and the second electrode / cathode of the analyte sensor and using a/the current signal in response to the applied modulated voltage signal to regulate the electric potential of the second electrode / working electrode, $E_A$, said electric potential of the second electrode / working electrode, $E_A$ will be driven towards / automatically reaches the sought-after electric potential working point $E_{wp}$.

Said electric potential working point $E_{wp}$ can then serve as reference potential for the analyte sensor.

Since the change in the current signal at the first electrode, i.e. the anode, with respect to changes in the electric potential of the first electrode in the plateau region or saturation region of the current-potential relation of the first electrode is smaller than the change in the current signal at the second electrode with respect to changes in the electric potential of the second electrode, i.e. the cathode, a regulation step of the applied modulated voltage signal as exemplary described above converges / moves towards an/the electric potential working point $E_{wp}$ in a reliable manner.

Furthermore, from the determined voltage level $V_L$ of the applied modulated voltage signal $V(t)$ that satisfies the above-identified condition, e.g. equation 3 and from the electric potential of/at the second electrode / cathode, an oxygen saturation / oxygen saturation level / oxygen concentration in the environment of the second electrode can be determined and outputted, e.g. outputted to a display.

For example, it is possible to use the oxygen contained in body fluids as cathode reaction. Then, the oxygen activity (which is closely correlated with its concentration) can be related to the cathode potential by the Nernst equation and in case of large current densities, extensions analogue to Butler-Volmer equation apply.

Then, after a calibration (at the factory or during use / operation of the analyte sensor), the change in cathode potential can be used to deduce the oxygen levels at the surface. Considering an oxygen transport model this can then enable the estimation of oxygen levels in the vicinity of the analyte sensor.

The information that can be derived from the measurement of an oxygen saturation / oxygen saturation level / oxygen concentration in the environment of the second electrode can inter alia be used to compensate for possible measurement errors in the analyte concentration to be measured.

It is further possible that energy that is released by the electrochemical reaction of the analyte with the first electrode can be harvested for supplying energy to the operation of the analyte sensor, e.g. can be supplied to an electric circuitry / electronics / battery / power source / processor of the analyte sensor.

This can increase the usage / operating time of the analyte sensor.

For example, provided the analyte reaction with the corresponding electron acceptor reaction has overall a negative free reaction enthalpy (i.e. occurs voluntarily), a part of this energy can be harvested. For example, the electrode current resulting from glucose oxidization can be accumulated in a capacitor and by means of a direct-current-to-direct-current (DCDC) conversion, the stored energy can be periodically transferred to an electrical consumer or storage unit.

Furthermore, the possible optional energy harvesting mechanism can also be used as means to realize the voltage signal modulation

An exemplary analyte to be measured by the analyte sensor can be glucose, i.e. the herein described method steps can be used to measure a/the concentration of glucose and the measured analyte concentration, e.g. the measured glucose concentration, can be outputted, for example on a display. For example, the analyte may be selected from the group consisting of glucose, cholesterol, ketones, triglycerides, and lactate. Additionally or alternatively, however, other types of analytes and/or any combination of analytes may be determined. Preferably, the analyte is glucose.

Herein the term glucose may be understood as relating to glucose measured by a herein described analyte sensor according to the herein described method steps in the interstitial fluid of a patient and/or to glucose measured in the blood of a patient, i.e. blood glucose.

An exemplary analyte sensor for measuring an analyte concentration, e.g. glucose concentration, can comprise a first electrode and a second electrode, wherein the first electrode can be configured to electrochemically react with the analyte to be measured for generating an electrical signal.

In particular, the analyte sensor can be configured for measuring an analyte concentration according to one, some or all of the above and herein exemplary method steps.

Furthermore, an/the exemplary analyte sensor may comprise no more than two electrodes, in particular no more than two separate electrodes.

Stated differently, an/the exemplary analyte sensor may comprise exactly two electrodes, i.e. exactly two separate electrodes.

However, a single electrode of an/the exemplary analyte sensor may have separate parts / components and may also be implemented as a combined electrode.

For example, the/said first electrode of said exemplary analyte sensor can be a/the working electrode, e.g. can act as anode during the electrochemical reaction with the analyte.

The second electrode can be an electrode selected from a group comprising the following types: a counter electrode and a combined counter/reference electrode.

As indicated earlier, no more than two separate electrodes are required to provide reliable and accurate measurements of an analyte with herein exemplary described analyte sensor.

In comparison with current analyte sensors, no additional separate dedicated reference electrode is needed.

In particular, the use of reference electrodes that are prone to undesired material wear and that can leak undesired material into their environments, such as, for example, Ag/AgCl (silver / silver chloride) reference electrode can be avoided in the herein described analyte sensor design.

The second electrode material may inter alia comprise a biocompatible material, such as, for example, gold or platinum.

The two-electrodes-only design of the herein described analyte sensor further allows a more cost-effective manufacturing and a more compact design of the analyte sensor.

To enable/generate and facilitate an/the electrochemical reaction of the analyte sensor with an analyte to be measured, the first electrode of an exemplary analyte sensor may comprise at least one enzyme. The first electrode may comprise only one enzyme or a mixture of two or more enzymes. Only one enzyme is preferred. Specifically, the enzyme is capable of catalyzing a chemical reaction converting the analyte, in particular glucose. Even more specifically the at least one enzyme is selected from the group consisting of a glucose oxidase (EC 1.1.3.4), a hexose oxidase (EC 1.1.3.5), an (S)-2 hydroxy acid oxidase (EC 1.1.3.15), a cholesterol oxidase (EC 1.1.3.6), a glucose dehydrogenase, a galactose oxidase (EC 1.1.3.9), an alcohol oxidase (EC 1.1.3.13), an L-glutamate oxidase (EC 1.4.3.11), and an L-aspartate oxidase (EC 1.4.3.16). In particular, the at least one enzyme is a glucose oxidase (GOx) and/or modifications thereof.

The at least one enzyme may be comprised in a sensing material. The term "sensing material", as used herein, specifically may refer, without limitation, to a material that may be or may comprise at least a polymeric material; specifically it may be or may comprise at least a polymeric material and at least a metal containing complex. The metal containing complex may be selected from the group consisting of transition metal element complexes, specifically the metal containing complex may be selected from osmium-complexes, ruthenium-complexes, vanadium-complexes, cobalt-complexes, and iron-complexes, such as ferrocenes, such as 2-aminoethylferrocene. Even more specifically, the sensing material may be a polymeric transition metal complex as described for example in WO 01/36660 A2, the content of which is included by reference. In particular, the sensing material may comprise a modified poly(vinylpyridine) backbone loaded with poly(bi-imidizyl) Os complexes covalently coupled through a bidentate linkage. A suitable sensing material is further described in Feldmann et al, Diabetes Technology & Therapeutics, 5 (5), 2003, 769-779, the content of which is included by reference. Suitable sensing materials further may include ferrocene-containing polyacrylamide-based viologen-modified redox polymer, pyrrole-2,2'-azino-bis(3-ethylbenzthiazoline-6-sulfonic acid)(ABTS)-pyrene, Naphthoquinone-LPEI. The polymeric transition metal complex may represent a redox mediator incorporated into a cross-linked redox polymer network. This is advantageous as it may facilitate electron transfer between the at least one enzyme or analyte and the conductive trace. In order to avoid a sensor drift, the redox mediator and the enzyme may be covalently incorporated into a polymeric structure.

In an embodiment the sensing material may comprise a polymeric material and $MnO_2$-particles or any other material catalyzing hydrogen peroxide oxidation reaction as well as the at least one enzyme. Another material catalyzing hydrogen peroxide oxidation reaction is Pt (platinum).

Moreover, the sensing material may additionally comprise at least one crosslinker; the crosslinker may for example be capable of crosslinking at least part of the sensing material.

As previously indicated, the second electrode can further be configured to measure an oxygen saturation / oxygen saturation level / oxygen saturation concentration in its environment.

Such an optional measurement of an oxygen saturation / oxygen saturation level / oxygen saturation concentration can be used to compensate for possible measurement errors of the analyte sensor, since the oxygen saturation / oxygen saturation level / oxygen saturation concentration can influence the electrochemical reaction used by the analyte sensor to measure the analyte concentration, in particular in the case of oxidative electrochemical reactions.

The optional possibility to measure and output an oxygen saturation / oxygen saturation level / oxygen saturation concentration in the blood and/or in interstitial fluids of patients may also be useful for further additional therapeutic and diagnostic purposes.

In an exemplary analyte sensor, the first and second electrode can be arranged on opposing sides of a/the substrate of the analyte sensor.

This exemplary design enables a more compact analyte sensor and more cost efficient manufacturing, since it is not necessary to have the conductive traces for both electrodes on the same side. For example, it is possible to cover a/the first side of a/the substrate with the conductive material of the first electrode / working electrode and a/the second side of a/the substrate with the conductive material of the second electrode / counter electrode and then apply further layers of materials such Ag/AgCl if necessary.

However, it is conceivable that other designs may be implemented for the analyte sensor, e.g. non-planar designs such as cylindrical designs, wherein a first electrode may be a rod electrode and the second electrode may be a cylindrical electrode that forms a sleeve around the second electrode, or wherein both electrodes are cylindrical with different diameters such that one cylindrical electrode can form a sleeve around the other.

An/the herein exemplary described analyte sensor may be implemented in a system for measuring an analyte concentration.

For example, an/the herein exemplary described analyte sensor may be part of a system comprising an implantable medical device, e.g. a continuous glucose monitoring system comprising an implantable medical device with a herein described analyte sensor.

The following figures illustrate exemplary the following aspects of the invention.

**Fig.1:** Exemplary schematic characteristic curve diagram for the relationship of current and electric potential of/in an exemplary analyte sensor

**Fig. 2:** Exemplary flow diagram of a method for measuring an analyte concentration using an electrochemical analyte sensor

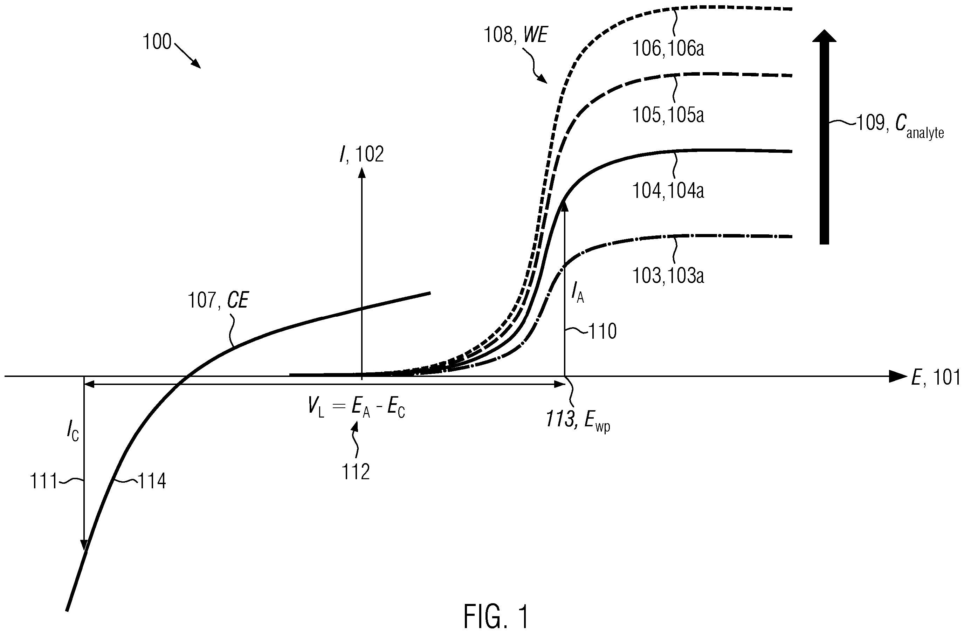

**Fig.1** exemplary shows a diagram 100 illustrating the characteristic curve(s) or characteristic line(s) 103, 104, 105, 106, 107 for the relationship of current (or current density), $I$(ordinate axis, 102), and electric potential (abscissa axis, 101), $E$, of/in an exemplary analyte sensor for the anodic and cathodic partial reaction(s) of the electrochemical conversion, e.g. via oxidation, of the analyte to be detected, e.g. glucose, with/at an enzyme, e.g. glucose oxidase, of the first electrode, i.e. the working electrode, of an exemplary analyte sensor.

In particular, curves or lines 103, 104, 105, and 106 show the behavior 108 of the relationship(s) of current (or current density) and electric potential at the first electrode, i.e. the working electrode, WE, or anode, of exemplary analyte sensor for different amounts or concentrations of the analyte to be detected, e.g. glucose.

Said curves or lines 103, 104, 105, and 106 inter alia illustrate that the exemplary oxidation reaction at the first electrode, i.e. the working electrode, saturates for sufficiently large electric potentials, as illustrated by the plateau regions 103a, 104a, 105a and 106a of said curves.

This is due to the fact that, at said sufficiently large electric electrode potentials, the amount / concentration of analyte, e.g. glucose, is not sufficiently high enough anymore to maintain the electrochemical conversion process / electrochemical reaction.

At sufficiently low or negative electric potentials of the first electrode, the electrochemical conversion process / electrochemical reaction comes to a standstill, i.e. the electrochemical conversion process / electrochemical reaction, e.g. oxidation, can be irreversible.

Curve 114 illustrates exemplary the behavior 107 for the relationship of current (or current density) and electric potential at the second electrode, e.g. a/the counter electrode, CE, or cathode, of the exemplary analyte sensor.

It is worthwhile noting that the behavior 107 for the relationship of current (or current density) and electric potential at the second electrode is essentially not affected or only affected by a negligible amount of the analyte concentration, i.e. the shape of the curve 114 is essentially not affected by the analyte concentration.

For a given voltage of an/the applied (modulated) voltage $V(t)$ or voltage level $V_L$, 112, between the two electrodes of the analyte sensor the amount or absolute value of the current at the first electrode is equal to the amount or absolute value of current at the second electrode.

Here, this is exemplified in the amount or absolute value of current, $I_A$, at the first electrode marked with reference sign 110 and that is equal to the amount or absolute value of current at the second electrode, $I_C$, marked with reference sign 111.

It is further noted that the exemplary index $A$ denotes the first electrode as anode and the exemplary index $C$ denotes the second electrode as cathode and that the applied voltage $V(t)$ or voltage level $V_L$, 112 can, as previously indicated, be expressed as:

$$V_L = E_A - E_C \qquad (5)$$

, wherein $E_A$ denotes the electric potential at the anode, e.g. at the second electrode, of the analyte sensor and $E_C$ denotes the electric potential at the cathode, e.g. at the first electrode, of the analyte sensor.

Using the further above exemplary described possible means and possible method steps for applying a modulated voltage signal between the first electrode and the second electrode of the/an analyte sensor, determining a current signal in response to the applied modulated voltage signal $V(t)$, an electric potential working point $E_{wp}$, 113, of the analyte sensor based on the determined current signal can be determined, wherein said determined electric potential working point $E_{wp}$, lies in a border region / border range of the electric potential of the analyte sensor / a border region / border range of the electric potential of the first electrode of the analyte sensor that is located just before a saturation plateau, 103a, 104a, 105a or 106a, e.g. just at or before the kink of curves 103, 104, 105, 106.

In particular, applying a/the modulated voltage signal $V(t)$ between the first electrode / working electrode / anode and the second electrode / cathode of the analyte sensor and determining a current signal in response to the applied modulated voltage signal, can allow to implicitly determine / set / reach / identify / obtain the desired electric potential working point $E_{wp}$, 113, of the analyte sensor.

For example, using the further above exemplary described step of regulating the applied modulated voltage signal $V(t)$ such that, for example, the determined current signal $I(t)$ fulfills a certain condition, e.g. the above-identified condition according to equation 3, the electric potential at the first electrode / working electrode / anode is driven towards the desired electric potential working point $E_{wp}$, 113.

In particular, as previously exemplary described, a possible condition to be fulfilled may be that the ratio of the determined amplitude of the current signal at the first electrode to the determined average current signal falls within a predetermined range, e.g. set by exemplary threshold values as indicated by equation 3, thereby determining / setting / reaching / obtaining / identifying an/the electric potential working point $E_{wp}$, 113, of the first electrode, that is close to the saturation region of the potential, i.e. just before a saturation plateau 103a, 104a, 105a or 106a, e.g. just at or before the kink of curves 103, 104, 105, 106.

The regulation of the applied modulated voltage signal can for example be carried out by using an analog or digital proportional-integral controller, e.g. a proportional-integral controller with $\log \dfrac{\hat{I}}{\langle I(t)\rangle}$ as input.

The thus determined / set / identified / obtained / reached electric potential working point $E_{wp}$, 113, of the analyte sensor / of the first electrode / of the working electrode / of the anode can then be used as reference electric potential for operating the analyte sensor.

In the shown example, the electric potential $E_A$ at the first electrode / anode / working electrode corresponds to the electric potential working point $E_{wp}$ that has been reached by applying a/the modulated voltage signal or voltage level $V_L$.

In particular, the thus determined electric potential working point $E_{wp}$, 113, of the analyte sensor can ensure that the electrical signal, i.e. the current signal, generated by the first electrode from the electrochemical reaction with the analyte is positively correlated to the concentration of the analyte to be measured, e.g. is directly proportional to the concentration of the analyte to be measured, e.g. glucose, and that, for example, this proportional relationship maintains a constant proportionality factor and is not affected by changes or drifts in electric potential of the analyte sensor during measurement operations of the analyte sensor.

Stated differently, the thus determined electric potential working point $E_{wp}$, 113, of the analyte sensor / of the working electrode of the analyte sensor can provide a more precise correlation of the electrical signal, e.g. the current signal, generated by the electrochemical reaction with respect to the concentration / concentration level(s) of an analyte to be measured. Hence, more precise measurements of the concentration / concentration level(s) of an analyte to be measured can be obtained.

For completeness, it is noted that the arrow marked with the reference sign 109 exemplary indicates increasing values for the concentration of an analyte, $C_{analyte}$, to be measured, e.g. glucose.

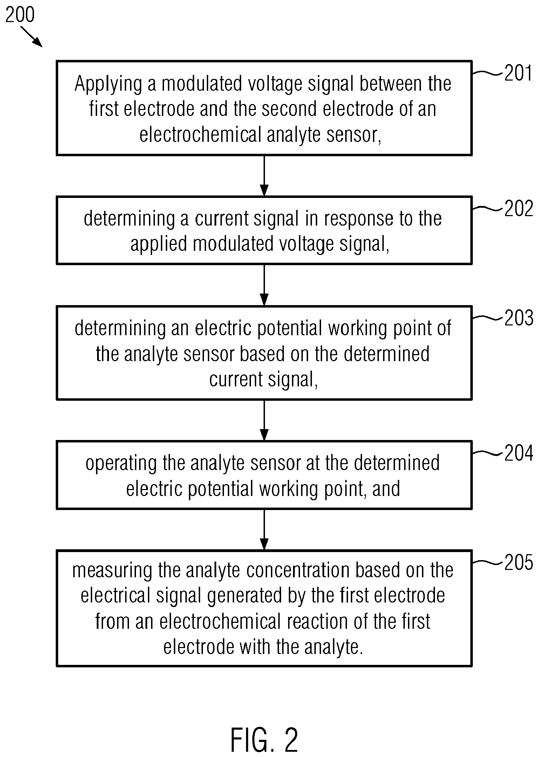

Fig. 2 shows an exemplary flow diagram for an exemplary method 200 for measuring an analyte concentration using an electrochemical analyte sensor, wherein the analyte sensor is comprising a first electrode and a second electrode, and the first electrode is being configured to react with the analyte for generating an electrical signal.

Therein a modulated voltage signal between the first electrode and the second electrode can be applied 201 and in response to the applied modulated voltage signal a current signal can be determined 202.

An electric potential working point of the analyte sensor can then be determined 203 based on the determined current signal.

The analyte sensor / the first electrode of the analyte sensor can then be operated 204 at the determined electric potential working point and the concentration of an analyte to be measured, e.g. glucose, can then be measured 205 based on / in dependence of the electrical signal, e.g. a current signal generated by the analyte sensor from the electrochemical reaction(s) of the analyte with the analyte sensor, i.e. with the electrodes of the analyte sensor.

Followed by Fig. 1 and Fig. 2, wherein the reference signs denote the following exemplary aspects, exemplary characteristics and exemplary steps.

| 100 | Exemplary characteristic curve diagram |
| 101 | Exemplary electric potential, exemplary abscissa axis |
| 102 | Exemplary electric current or current density, exemplary ordinate axis |

(continued)

| 103 | Exemplary characteristic curve or line for relationship of electric current or current density to electric potential at first electrode / working electrode / anode at an exemplary first concentration level of analyte |
| --- | --- |
| 103a | Exemplary plateau of curve 103 marking an exemplary region / range where the electrochemical reaction, e.g. an oxidation, of the analyte with the analyte sensor is saturated |
| 104 | Exemplary characteristic curve or line for relationship of electric current or current density to electric potential at first electrode / working electrode / anode at an exemplary second concentration level of analyte |
| 104a | Exemplary plateau of curve 104 marking an exemplary region / range where the electrochemical reaction, e.g. an oxidation, of the analyte with the analyte sensor is saturated |
| 105 | Exemplary characteristic curve or line for relationship of electric current or current density to electric potential at first electrode / working electrode / anode at an exemplary third concentration level of analyte |
| 105a | Exemplary plateau of curve 105 marking an exemplary region / range where the electrochemical reaction, e.g. an oxidation, of the analyte with the analyte sensor is saturated |
| 106 | Exemplary characteristic curve or line for relationship of electric current or current density to electric potential at first electrode / working electrode / anode at an exemplary fourth concentration level of analyte |
| 106a | Exemplary plateau of curve 106 marking an exemplary region / range where the electrochemical reaction, e.g. an oxidation, of the analyte with the analyte sensor is saturated |
| 107 | Exemplary behavior of second electrode / counter electrode, CE, / cathode |
| 108 | Exemplary behavior of first electrode / working electrode, WE, / anode |
| 109 | Exemplary direction marking an increase of concentration / concentration levels of an analyte to be measured, e.g. glucose |
| 110 | Exemplary current / current density / current signal at first electrode / working electrode, WE, / anode |
| 111 | Exemplary current / current density / current signal at second electrode / counter electrode, WE, / cathode |
| 112 | Exemplary applied voltage V(t) or voltage level $V_L$ |
| 113 | Exemplary electric potential working point $E_{wp}$ |
| 114 | Exemplary characteristic curve or line for relationship of electric current or current density to electric potential at the second electrode / counter electrode, CE, / cathode |
| 200 | Exemplary flow chart for exemplary method for measuring an analyte concentration using an electrochemical analyte sensor |
| 201, 202, 203, 204, 205 | Exemplary method steps |

**Claims**

1. Method (200) for measuring an analyte concentration using an electrochemical analyte sensor, the analyte sensor comprising a first electrode and a second electrode, the first electrode being configured to react with the analyte for generating an electrical signal, the method comprising:

    applying (201) a modulated voltage signal between the first electrode and the second electrode,
    determining (202) a current signal in response to the applied modulated voltage signal,
    determining (203) an electric potential working point of the analyte sensor based on the determined current signal,
    operating (204) the analyte sensor at the determined electric potential working point, and
    measuring (205) the analyte concentration based on the electrical signal generated by the first electrode.

2. Method according to the preceding claim, wherein the modulated voltage signal applied between the first electrode and the second electrode is applied in time-discrete steps.

3. Method according to one of the preceding claims, wherein the step of determining a current signal in response to the applied modulated voltage signal comprises:
determining the amplitude of the current signal and the average current signal at the first electrode in response to the applied modulated voltage signal and in response to the electrical signal generated at the first electrode from the reaction with the analyte.

4.  Method according to the preceding claim, wherein the step of determining an/the electric potential working point of the analyte sensor based on the determined current signal comprises:
    regulating the applied modulated voltage signal such that the ratio of the determined amplitude of the current signal at the first electrode to the determined average current signal falls within a predetermined range to determine an electric potential working point of the first electrode.

5.  Method according to one of the preceding claims, wherein the first electrode is a working electrode and/or wherein the second electrode is selected from a group comprising the following types: a counter electrode and a combined counter/reference electrode.

6.  Method according to one of the preceding claims, wherein energy released by the electro-chemical reaction of the analyte with the first electrode is harvested for supplying energy to the operation of the analyte sensor.

7.  Method according to one of the preceding claims, wherein the analyte is glucose and wherein the concentration of glucose is measured and/or wherein the measured analyte concentration is outputted on a display.

8.  Analyte sensor for measuring an analyte concentration, the analyte sensor comprising a first electrode and a second electrode, the first electrode being configured to electrochemically react with the analyte for generating an electrical signal and the analyte sensor being configured for measuring an analyte concentration according to one of the preceding claims.

9.  Analyte sensor according to the preceding claim, wherein the first electrode is a working electrode and/or wherein the second electrode is an electrode selected from a group comprising the following types: a counter electrode and a combined counter/reference electrode and/or wherein the second electrode comprises gold or platinum.

10. Analyte sensor according to one of the preceding sensor claims, wherein the first electrode comprises at least one of an enzyme, e.g. glucose oxidase, and/or a polymeric transition complex, e.g. a transition metal complex selected from osmium-complexes, ruthenium-complexes, vanadium-complexes, cobalt-complexes, or iron-complexes, such as ferrocenes, e.g. 2-aminoethylferrocene.

11. Analyte sensor according to the preceding claim, the first electrode comprising at least one transition metal complex comprising a modified poly(vinylpyridine) backbone loaded with poly(bi-imidizyl) Os complexes covalently coupled through a bidentate linkage.

12. Analyte sensor according to one of the preceding sensor claims, wherein the second electrode is configured to measure an oxygen saturation in the environment of the second electrode.

13. Analyte sensor according to one of the preceding sensor claims, wherein the first and second electrode are arranged on opposing sides of a/the substrate of the analyte sensor.

14. System for measuring an analyte concentration comprising an analyte sensor according to one of the preceding sensor claims.

15. System according to the preceding claim, the system comprising an implantable medical device with an analyte sensor according to one of the preceding sensor claims.

FIG. 1

EP 4 134 004 A1

200

Applying a modulated voltage signal between the first electrode and the second electrode of an electrochemical analyte sensor, ~201

determining a current signal in response to the applied modulated voltage signal, ~202

determining an electric potential working point of the analyte sensor based on the determined current signal, ~203

operating the analyte sensor at the determined electric potential working point, and ~204

measuring the analyte concentration based on the electrical signal generated by the first electrode from an electrochemical reaction of the first electrode with the analyte. ~205

FIG. 2

Europäisches
Patentamt

European
Patent Office

Office européen
des brevets

# EUROPEAN SEARCH REPORT

**Application Number**

EP 21 19 0800

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/213057 A1 (FELDMAN BENJAMIN [US] ET AL) 26 August 2010 (2010-08-26) | 1,2,5-15 | INV. A61B5/1486 |
| A | * claims 47,50 * <br> * abstract * <br> * figures * <br> * paragraphs [0027] - [0143] * <br> ----- | 3,4 | G01N27/327 <br> G01N27/403 <br> G01N27/413 <br> G01N27/48 <br> G01N27/49 |
| A | US 2010/147707 A1 (LIU JIANYUN [CN] ET AL) 17 June 2010 (2010-06-17) <br> * abstract * <br> * figures * <br> * paragraphs [0029] - [0030] * <br> ----- | 1-15 | |
| A | US 4 798 655 A (DIAMOND HOWARD [US]) 17 January 1989 (1989-01-17) <br> * the whole document * <br> ----- | 1-15 | |

| | |
|---|---|
| | **TECHNICAL FIELDS SEARCHED (IPC)** |
| | G01N <br> A61B |

The present search report has been drawn up for all claims

| Place of search | Date of completion of the search | Examiner |
|---|---|---|
| Munich | 26 January 2022 | Ruchaud, Nicolas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................................

& : member of the same patent family, corresponding
    document

EPO FORM 1503 03.82 (P04C01)

## EP 4 134 004 A1

### ANNEX TO THE EUROPEAN SEARCH REPORT
### ON EUROPEAN PATENT APPLICATION NO.

EP 21 19 0800

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2022

| Patent document cited in search report | | Publication date | Patent family member(s) | | Publication date |
|---|---|---|---|---|---|
| US 2010213057 | A1 | 26-08-2010 | EP | 2401390 A1 | 04-01-2012 |
| | | | EP | 3255154 A1 | 13-12-2017 |
| | | | US | 2010213057 A1 | 26-08-2010 |
| | | | US | 2010213082 A1 | 26-08-2010 |
| | | | US | 2017188904 A1 | 06-07-2017 |
| | | | US | 2018296139 A1 | 18-10-2018 |
| | | | WO | 2010099335 A1 | 02-09-2010 |
| US 2010147707 | A1 | 17-06-2010 | NONE | | |
| US 4798655 | A | 17-01-1989 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0136660 A2

**Non-patent literature cited in the description**

- **FELDMANN et al.** *Diabetes Technology & Therapeutics,* 2003, vol. 5 (5), 769-779